# EUROPEAN PATENT APPLICATION

(11) **EP 4 306 108 A1**
(43) Date of publication of application: **17.01.2024**
(21) Application number: 22386045.3
(22) Date of filing: 11.07.2022
(51) Int. Cl.: A61K 31/197, A61P 35/00

(54) **5-AMINOLEVULINIC ACID, OR AN ESTER THEREOF FOR USE IN TREATMENT OF CANCER BASED ON THE INHIBITION OF LACTATE DEHYDROGENASE**

(71) Applicant: Theodossiou, Theodossis, 0274 Oslo (NO)
(72) Inventor: Theodossiou, Theodossis, 0274 Oslo (NO); Grigalavicius, Mantas, 346 Gjettum (NO); Henriksen Raabe, Tine Therese, 0655 Oslo (NO); Ezzatpanah, Somayeh, 0494 Oslo (NO)
(74) Representative: Roukounas, Dimitrios

(57) **Abstract**

Use of 5-aminolevulinic acid, or an ester, or a pharmaceutically acceptable salt thereof, in the treatment of cancer through the inhibition of lactate dehydrogenase in cancer cells.

## Description

### FIELD OF THE INVENTION

The present invention relates to the use of 5-aminolevulinic acid, or an ester, or a pharmaceutically acceptable salt thereof, in the treatment of cancer.

### BACKGROUND OF THE INVENTION

5-Aminolevulinic acid (5-ALA), also known as δ-aminolevulinic acid is a natural, non-proteinogenic δ-aminoacid having the following chemical structure:

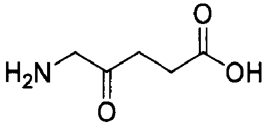

In non-photosynthetic eukaryotes, 5-ALA is produced through the condensation of glycine and succinyl-CoA, catalyzed by the mitochondrial 5-ALA synthase (ALAS)( Kikuchi, G., Kumar, A., Talmage, P. & Shemin, D. The enzymatic synthesis of delta-aminolevulinic acid. J Biol Chem 233, 1214-1219 (1958)). 5-ALA is the first committed step in the biosynthesis of heme, leading to the final precursor of heme, protoporphyrin IX (PpIX), which is a very potent endogenous photosensitizer (PS) used in photodynamic therapy of cancer (PDT)(Macrobert, A. J. & Theodossiou, T. in Encyclopedia of Modern Optics Vol. 1 (ed R. D. Guenther) 53-62 (Elsevier, Amsterdam, 2005; Agostinis, P. et al. Photodynamic therapy of cancer: an update. CA Cancer J Clin 61, 250-281, doi:10.3322/caac.20114 (2011)). The potential of PpIX as a PS was uncovered by Kennedy and Pottier (Kennedy, J. C. & Pottier, R. H. Endogenous protoporphyrin IX, a clinically useful photosensitizer for photodynamic therapy. J Photochem Photobiol B 14, 275-292, doi:10.1016/1011-1344(92)85108-7 (1992)), who used topical administration of high, exogenous 5-ALA concentrations. This overrode the normal feedback inhibition of 5-ALA synthase by heme and consequently overloaded heme biosynthesis ultimately achieving high protoporphyrin IX accumulation in tumours. This cancer specificity can be attributed to differences in the porphyrin biosyntheric pathway activity in tumours versus normal tissues (Batlle, A. M. Porphyrins, porphyrias, cancer and photodynamic therapy--a model for carcinogenesis. J Photochem Photobiol B 20, 5-22, doi:10.1016/1011-1344(93)80127-u (1993)), including i) lack of ferrochelatase in tumours (Ohgari, Y. et al. Mechanisms involved in delta-aminolevulinic acid (ALA)-induced photosensitivity of tumor cells: relation of ferrochelatase and uptake of ALA to the accumulation of protoporphyrin. Biochem Pharmacol 71, 42-49, doi:10.1016/j.bcp.2005.10.019 (2005)) (the enzyme responsible for the insertion of Fe into PpIX to convert it into heme), ii) reduced availability of Fe in tumour cells (Krieg, R. C., Fickweiler, S., Wolfbeis, O. S. & Knuechel, R. Cell-type specific protoporphyrin IX metabolism in human bladder cancer in vitro. Photochem Photobiol 72, 226-233, doi:10.1562/0031-8655(2000)072<0226:ctspim>2.0.co;2 (2000)) to catalyze the PpIX - heme conversion, and iii) increased activity of enzymes (Navone, N. M., Polo, C. F., Frisardi, A. L., Andrade, N. E. & Battle, A. M. Heme biosynthesis in human breast cancermimetic "in vitro" studies and some heme enzymic activity levels. Int J Biochem 22, 1407-1411, doi:10.1016/0020-711×(90)90230-z (1990); Hinnen, P. et al. Biochemical basis of 5-aminolaevulinic acid-induced protoporphyrin IX accumulation: a study in patients with (pre)malignant lesions of the oesophagus. Br J Cancer 78, 679-682, doi:10.1038/bjc.1998.559 (1998)), e.g. porphobilinogen deaminase, that promote PpIX synthesis in cancer cells.

The specific accumulation of PpIX in cancer cells has found applications outside the obvious in the field of PDT (Peng, Q. et al. 5-Aminolevulinic acid-based photodynamic therapy. Clinical research and future challenges. Cancer 79, 2282-2308, doi:10.1002/(sici)1097-0142(19970615)79:12<2282::aid-cncr2>3.0.co;2-o (1997)): 5-ALA-derived PpIX photodynamic diagnosis has already been clinically applied for the diagnosis of bladder (Inoue, K. et al. Comparison between intravesical and oral administration of 5-aminolevulinic acid in the clinical benefit of photodynamic diagnosis for nonmuscle invasive bladder cancer. Cancer 118, 1062-1074, doi:10.1002/cncr.26378 (2012)), prostate (Fukuhara, H. et al. Photodynamic diagnosis of positive margin during radical prostatectomy: preliminary experience with 5-aminolevulinic acid. Int J Urol 18, 585-591, doi:10.1111/j.1442-2042.2011.02789.x (2011)) and brain (Stummer, W. et al. Intraoperative detection of malignant gliomas by 5-aminolevulinic acid-induced porphyrin fluorescence. Neurosurgery 42, 518-525; discussion 525-516, doi:10.1097/00006123-199803000-00017 (1998)) malignancies. In fact, 5-ALA was approved by the U.S. Food and Drug Administration (FDA) in 2017, as an adjunct for the visualization of malignant tissue, in grade III or IV glioma (NDA 208630/SN0014), and is currently used in the clinic to precisely guide the resection of brain cancers such as malignant glioma and glioblastoma multiforme (GBM) (Stummer, W. et al. Fluorescence-guided resection of glioblastoma multiforme by using 5-aminolevulinic acid-induced porphyrins: a prospective study in 52 consecutive patients. J Neurosurg 93, 1003-1013, doi:10.3171/jns.2000.93.6.1003 (2000); Stummer, W. et al. Fluorescence-guided surgery with 5-aminolevulinic acid for resection of malignant glioma: a randomised controlled multicentre phase III trial. Lancet Oncol 7, 392-401, doi:10.1016/S1470-2045(06)70665-9 (2006)). The accumulation of 5-ALA-derived PpIX in brain lesions in particular is further assisted by the local destruction of the blood brain barrier (BBB) by the neoplasia infiltration allowing the free influx of 5-ALA, which has otherwise been found to exhibit very low permeability of the intact BBB due to a saturable efflux mechanism at the choroid plexus (Ennis, S. R. et al. Transport of 5-aminolevulinic acid between blood and brain. Brain Res 959, 226-234, doi:10.1016/s0006-8993(02)03749-6 (2003)).

Lactate dehydrogenase (LDH) is a key glycolytic enzyme that catalyzes the final step in glycolysis (Melkonian, E. A. & Schury, M. P. Biochemistry, Anaerobic Glycolysis. (StatPearls Publishing, Treasure Island (FL), 2020)), reducing pyruvate to lactate and regenerating NAD+ necessary for continued glycolysis. Expression of the LDH-A gene is upregulated in many cancers to support the elevated glycolytic activity in these cells (Warburg effect) (Liberti, M. V. & Locasale, J. W. The Warburg Effect: How Does it Benefit Cancer Cells? Trends Biochem Sci 41, 211-218, doi:10.1016/j.tibs.2015.12.001 (2016); Feng, Y. et al. Lactate dehydrogenase A: A key player in carcinogenesis and potential target in cancer therapy. Cancer Med 7, 6124-6136, doi:10.1002/cam4.1820 (2018)). Disruption of the glycolysis by inhibition of LDH can prove fatal to cancer cells but not to normal cells which, in principle, are able to compensate with an increase in oxidative phosphorylation-produced ATP (Feng, Y. et al. Lactate dehydrogenase A: A key player in carcinogenesis and potential target in cancer therapy. Cancer Med 7, 6124-6136, doi:10.1002/cam4.1820 (2018); Miao, P., Sheng, S., Sun, X., Liu, J. & Huang, G. Lactate dehydrogenase A in cancer: a promising target for diagnosis and therapy. IUBMB Life 65, 904-910, doi:10.1002/iub.1216 (2013)). Indeed, inhibition of LDH reintroduces oxidative stress leading to inhibition of tumor progression (Le, A. et al. Inhibition of lactate dehydrogenase A induces oxidative stress and inhibits tumor progression. Proc Natl Acad Sci U S A 107, 2037-2042, doi:10.1073/pnas.0914433107 (2010)). Since LDH is a cytosolic enzyme present in most eukaryotic cells, its release through compromised plasma membranes is routinely used in the lab as an assay of cell death, by necrosis (Chan, F. K., Moriwaki, K. & De Rosa, M. J. Detection of necrosis by release of lactate dehydrogenase activity. Methods Mol Biol 979, 65-70, doi:10.1007/978-1-62703-290-2_7 (2013)). Apart from the laboratory setting, however, LDH is a useful clinical diagnostic marker (Khan, A. A., Allemailem, K. S., Alhumaydhi, F. A., Gowder, S. J. T. & Rahmani, A. H. The Biochemical and Clinical Perspectives of Lactate Dehydrogenase: An Enzyme of Active Metabolism. Endocr Metab Immune Disord Drug Targets 20, 855-868, doi:10.2174/1871530320666191230141110 (2020)) in cancer (Forkasiewicz, A. et al. The usefulness of lactate dehydrogenase measurements in current oncological practice. Cell Mol Biol Lett 25, 35, doi:10.1186/s11658-020-00228-7 (2020)) and many other pathologies. Furthermore, LDH is amongst the most sought-after targets for inhibition as part of an anticancer strategy.

### SUMMARY OF INVENTION

The present invention provides 5-ALA, an ester, or a pharmaceutically acceptable salt thereof for use in the treatment of cancer by inhibiting the activity of LDH in cancer cells.

The present inventors have found that the compounds of the present invention are inhibitors of LDH and that they can be used in the treatment of cancer by inhibiting the terminal conversion of pyruvate to Lactate in cancer cells.

The present invention provides also a pharmaceutical composition comprising 5-ALA, or an ester, or a pharmaceutically acceptable salt thereof for use in the treatment of cancer by inhibiting the activity of LDH in cancer cells.

### BRIEF DESCRIPTION OF THE DRAWINGS

Figure 1 shows the reduction in the glycolytic activity of U87, T98G, LN18 and M059K cells, following the injection of 5-ALA in real time in seahorse metabolic assays.
Figure 2 shows the changes in ATP production from either respiration or glycolysis in human glioblastoma cells upon addition of 1000µM of 5-ALA. In all cases the bottom bars represent control conditions while the top bars represent respiration produced ATP (white) and glycolysis produced ATP (shaded with diagonal lines), after addition of 1mM 5-ALA. A. U87 cells; B, T98G cells; C. M059K cells and D. LN18 cells
Figure 3 shows the results of LDH inhibition in enzyme solutions (5µg/mL) by 5-ALA. A. no enzyme; B. LDH and 5-ALA; C. LDH.
Figure 4 shows the results of LDH inhibition in human glioblastoma cells (cell lysates) by 5-ALA. A. U87; B. T98G; C. LN18 and D. M059K.
Figure 5 shows the cytotoxic effect of 5-ALA induced LDH inhibition on human glioblastoma cells.

### DETAILED DESCRIPTION OF THE INVENTION

The present invention provides a compound of formula (I) or a pharmaceutically acceptable salt thereof 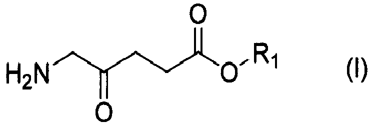 wherein R₁ is hydrogen, or a C₁-C₆ aklyl group,
for use in the treatment of cancer in a patient by inhibiting the activity of LDH in cancer cells.

Preferably, R₁ is hydrogen, or a C₁-C₃ aklyl group. More preferably, R₁ is hydrogen.

Preferably, the patient is a human patient.

The prior art discloses the use of 5-ALA in the photodynamic detection of cancer and in the treatment of cancer by photodynamic therapy. In these approaches, 5-ALA is endogenously converted into protoporphyrin IX (PpIX), which then acts as photosensitizer.

The present inventors have surprisingly found that a compound of formula (I), or a pharmaceutically acceptable salt thereof, is an inhibitor of LDH, a key enzyme in the glycolytic activity of cells.

It is known that the disruption of glycolysis can prove fatal to cancer cells. Namely, one of the main hallmarks of cancer is the reprogramming of cancer cell metabolism, to increase their chances for survival, proliferation and growth. This reprogramming, typically means shift to a predominantly glycolytic phenotype with a simultaneous suppression of cell respiration, also known as the Warburg effect, however such reprogramming varies across the different cancer cells with some of them even maintaining a high respiration. Acids like lactate which end up in the extracellular space, can decrease the pH of the tumour microenvironment, promote tumour invasion and even lead to the suppression of anticancer, professional killer cells. As a consequence of the insidious hypoxia in many tumours, particularly as they grow bigger, the hypoxia inducible factor (HIF-1) is upregulated. This in turn activates several glycolytic enzyme genes, including LDH, which participates in the redox cycle of NADH/NAD+, as it recycles NAD+ from NADH, linked to the reduction of pyruvate to lactate.

Upon inhibition of LDH, the above advantages of cancer cells are taken away, forcing the cells to resort to respiration for their energy needs. This sudden switch back to a respiratory metabolism has serious repercussions for the tumour cells, which in principle have mitochondria adapted to the glycolytic profile: It can lead to severe oxidative stress, and since, in many cases, the antioxidant defenses may be decreased as they are not needed in a predominantly glycolytic metabolism, this can lead to cytostasis and cell death.

Thus, the present invention provides a treatment of cancer in which a compound of formula (I), or a pharmaceutically acceptable salt thereof, is used to inhibit the activity of LDH in cancer cells which leads to cytostasis and death of the cancer cells. Importantly, the present inventors have found that the inhibition of LDH is achieved by a compound of formula (I) itself, i.e., by 5-ALA, or an ester, or a pharmaceutically acceptable salt thereof and not by other compounds endogenously produced in the biosynthesis of heme, such as PpIX. Thus, the treatment according to the present invention differs from treatments of the prior art in which 5-ALA, is used in photodynamic therapy. Nevertheless, photodynamic therapy, as well as other cancer treatments, such as chemotherapy, can be used as adjuvant therapies following or at any point during the treatment of the present invention.

Disorders or abnormalities which may be treated include malignant cancer conditions, such as cancerous growths or tumours, and their metastases; tumours such as sarcomas and carcinomas, in particular solid tumours. The invention is particularly suitable for the treatment of tumours, especially those which are located below the surface of the skin, i.e. internal cancers or deeply-sited cancers.

The present invention can be applied to cancer cells which exhibit increased glycolytic activity, but it can also be applied to cancer cells which do not exhibit increased glycolytic activity. Examples of cancers which can be treated with the present invention include brain cancer, sarcomas, including osteogenic and soft tissue sarcomas; carcinomas, e.g. breast, lung, cerebral, bladder, thyroid, prostate, colon, rectum, pancreas, stomach, liver, liver duct, uterine, hepatic, renal, prostate, cervical and ovarian carcinomas; lymphomas, including Hodgkin and non-Hodgkin lymphomas; neuroblastoma, melanoma, myeloma, Wilm's tumour; leukemias, including acute lymphoblastic leukaemia and acute myeloblastic leukaemia; astrocytomas, gliomas, mixed gliomas and retinoblastomas; mesothelioma. The invention finds particular value in the treatment of deep lying cancerous lesions that are difficult to access non-invasively. Thus, preferably, the cancer to be treated according to the present invention is brain cancer, such as malignant glioma and glioblastoma multiforme, astrocytomas, mixed gliomas, and other difficult to cure cancers, such as pancreas, liver, liver duct, cervical, mesothelioma, colon and lung.

According to a preferred embodiment, the cancer according to the present invention is a brain cancer, preferably gliomas and astrocytomas like glioblastoma multiforme. A key factor which determines the high stockpile of 5-ALA in the brain cancer lesions, is the fact that intact BBB (normal brain tissue) is not permeable to 5-ALA. In contrast, compromised BBB (in and around the GBM lesions) can facilitate the influx and local build-up of 5-ALA.

The pharmaceutically acceptable salt of a compound of formula (I) can be, for example, a pharmaceutically acceptable acid addition salt of an inorganic or organic acid. Examples of inorganic acid addition salts include hydrochloride, hydrobromide, sulfate, nitrate, and phosphate. Examples of organic acid addition salts include acetate, lactate, citrate, tartrate, succinate, maleate and fumarate. When R₁ in formula (I) is hydrogen, the pharmaceutically acceptable salt can also be a base addition salt of an inorganic or organic base, such as sodium hydroxide, potassium hydroxide, ammonium hydroxide, N,N-dimethylethanolamine, L-Lysine, L-arginine and the like. The pharmaceutically acceptable salts can be produced by methods well known in the art.

Preferably, the compound of formula (I) is 5-aminolevulinic acid hydrochloride or 5-aminolevulinic acid phosphate.

The compound of formula (I) of a pharmaceutically acceptable salt thereof is typically administered as a pharmaceutical composition.

Thus, the present invention provides also a pharmaceutical composition comprising as active ingredient an LDH inhibitor for use in the treatment of cancer, wherein the LDH inhibitor is a compound of formula (I) or a pharmaceutically acceptable salt thereof, as defined above.

The composition may be formulated, for example, for parenteral, intramuscular, intracerebral, intravenous, intraperitoneal, subcutaneous, transdermal/intradermal, intratumoural, intrarectal, oral, inhalation administration or by infusion, for example, via a drip. The composition may have different forms, such as solid or liquid forms, for example it can have the form of powder, tablet, capsule, suppository, solution, suspension, emulsion, gel, cream, ointment, spray, transdermal patch, or lotion. The composition typically comprises a compound of formula (I), or a pharmaceutically acceptable salt thereof as active ingredient and a pharmaceutically acceptable carrier. The pharmaceutically acceptable carrier may comprise one or more excipients, such as solvents, buffering agents, emulsifying agents, preservatives, antioxidants, diluents, binders and the like, which are well known in the art. Examples of solvents, include saline, water, phosphate buffered saline, ethanol, isopropyl alcohol and mixtures thereof. Examples of buffering agents include citric acid monohydrate, acetic acid, sodium acetate and sodium hydrogen phosphate. Examples of emulsifying agents include sodium lauryl sulfate, lecithin, polysorbate and sorbitan monooleate. Examples of preservatives include sodium benzoate, benzyl alcohol and parahydroxy benzoic acids and their alkyl esters. Examples of antioxidants include sodium metabisulfite, butylated hydroxyanisole, butylated hydroxytoluene and ascorbic acid. Examples of diluents include lactose, sucrose, dextrose, mannitol and sorbitol. Examples of binders include hydroxypropylcellulose, hydroxyethylcellulose, dextrose, xylitol, polyvinylpyrrolidone and polyethylene glycol.

When the composition is in the form of an aqueous liquid, the pH of the composition is preferably from 2 to 9, more preferably from 4.5 to 6.5.

According to a preferred embodiment, the treatment of cancer according to the present invention involves the sustained delivery of the compound of formula (I), or a pharmaceutically acceptable salt thereof, to a patient. The sustained delivery can be achieved by various methods, well known in the art. For example, the compound of formula (I) can be formulated in a sustained release drug delivery system. Such delivery systems are well known in the art and include, for example, compositions for oral, parenteral or topical use. In another example, sustained delivery can be obtained by continuous administration of a composition comprising the compound, such as by infusion via a drip.

The sustained delivery ensures that the compound of formula (I), or a pharmaceutically acceptable salt thereof, will reach and maintain stable levels in the lesion so that it can exert its inhibitory action on LDH of target cells. The compound should typically be delivered over a period of several hours, or over longer periods, such as several days or several weeks. Preferably. the duration of the delivery is from 12 hours to one month. More preferably, the duration of delivery is from 1 day to 20 days.

In a preferred embodiment, the compound of formula (I) or a pharmaceutically acceptable salt thereof may be formulated in the form of a thermoresponsive composition which becomes a thermogel at physiological temperature (i.e. once delivered to the body). The composition can optimally release its load over a period of up to 24 hours, e.g. 5 to 24 hours, and then the administration of the composition can be repeated, e.g. 1-30 days. The use of temperature-responsive polymers allows the formulation of low viscosity solutions which are suitable for subcutaneous injection and which, in response to body temperature, undergo an *in situ* phase-transition and turn into a gel. To optimise the thermosetting properties of the gel-network different polymers and their copolymers may be used. Such polymer materials are known and used in the art and include, for example: poly(lactic-co-glycolic acid) (PLGA); alginate/hyaluronic acid; poly(N-isopropylacrylamide); and poloxamers.

In another preferred embodiment, nanoparticles and/or microparticles containing a compound of formula (I) or a pharmaceutically acceptable salt thereof may also be used in order to provide a controlled and continuous release of the active ingredient over a prolonged period, of up to 24 hours, e.g. 5 to 24 hours, and then the administration of the composition can be repeated, e.g. for 1 to 30 days. Examples of such carriers include (i) micellar carriers, (ii) liposomes, (iii) dendrimeric or polymeric nanocarrriers, and (iv) solid lipid nanoparticles. Any such particles may be included in the compositions herein described such that these form a reservoir for release of the active ingredient *in situ.*

When the treatment of cancer according to the present invention includes sustained delivery of a compound of formula (I) or a pharmaceutically acceptable salt thereof, the delivered amount is preferably such that the concentration of 5-ALA in the patient's blood in the course of the treatment is from 0.05mM (mmol/L) to 50mM, more preferably from 0.1mM to 40mM, even more preferably from 0.1mM to 20mM, even more preferably from 0.1mM to 10mM, even more preferably from 0.1mM to 5mM and most preferably from 0.5mM to 5mM. In this way, the blood concentration of the active ingredient is maintained for longer periods, for example, ranging from 12 hours to one month, and the active ingredient can exert its inhibitory action on the target cell LDH activity for long enough to irreversibly destroy the target cancer cells. The concentration of 5-ALA in the patient's blood can be measured following methods well known in the art, such as chromatography, spectroscopy etc.

The composition of the present invention can be administered, for example, parenterally, intramuscularly, intracerebrally, intravenously, subcutaneously, transdermally, intratumourally, intraperitoneally, intrarectally, orally, by inhalation, or by infusion via a drip.

The composition may be administered systemically (e.g. orally or parenterally), or alternatively it may be locally applied (e.g. topically) at or near the affected site. The route of administration will depend on various factors, such as the severity, nature and location of the disease to be treated.

The frequency of administration and the administered dosage of a compound of formula (I), or a pharmaceutically acceptable salt thereof, depend on various factors, such as the type of cancer, the route of administration and the form of the composition comprising the compound. Thus, cancer cells that exhibit a Warburg phenotype, can be typically treated with a lower dose, and for a shorter period. Cancer cells that do not exhibit a Warburg phenotype require typically higher doses administered over a longer period. In the case of GBM resection guidance, 5-ALA is administered as a single bolus and the fluorescence of the resulting PpIX is used intraoperatively. By contrast, LDH inhibition from that single 5-ALA bolus is not long enough to elicit a curative effect, due to the fast systemic metabolism of 5-ALA and its conversion to PpIX .

The present invention provides a treatment of cancer through the inhibition of the activity of LDH in cancer cells, leading to stasis and/or death of such cells, providing thereby a simple solution in the treatment of cancer. Furthermore, the present invention enables the targeting of cancer cells without affecting normal cells, which are, in principle, able to compensate the inhibition of LDH with an increase in oxidative phosphorylation-produced ATP.

### EXAMPLES

The cells used in the present examples were human glioblastoma multiforme (GBM) cell lines, purchased from the American Type Culture Collection (ATCC, Manassas, VA, USA). These cell lines were LN18 (ATCC^{®}, CRL-2610^{™}), U87 (ATCC^{®}, HTB-14^{™}), T98G (ATCC^{®}, CRL-1690^{™}) and M059K (ATCC^{®}, CRL-2365^{™}). All cells were grown in RPMI 1640 without phenol red, supplemented with 10% fetal bovine serum (FBS) and penicillin/streptomycin at 37 °C in a 5% CO2 and 95% humidified atmosphere.

### Example 1

This example shows the effect of the in-situ addition of 5-ALA on the metabolic profile of four GBM cell lines (U87, T98G, MO59K and LN18).

In order to study the changes in cell metabolism in GBM cells following the in-situ addition of 5-ALA, the Seahorse XFe96 analyzer (Agilent Technologies) was employed. Briefly, 4×104 cells were seeded in a seahorse plate and incubated overnight in complete RPMI 1640 medium at 37 °C, and a 5% CO2 humidified atmosphere. One hour prior to the experiments, the cells had their media changed to unbuffered XF RPMI pH 7.4 medium supplemented with 10 mM glucose, 2 mM L-glutamine, and 2 mM sodium pyruvate and were incubated for one hour at 37 °C, 0% CO2 atmosphere. The basal oxygen consumption and media acidification rates (OCR and ECAR respectively) of the cell groups were measured in four cycles. After those four cycles injections of i) 5-ALA (500 - 1000 µM), ii) Oligomycin A (OLIGO) 1 µM, iii) Carbonyl cyanide 4-(trifluoromethoxy)phenylhydrazone (FCCP) 1 µM and iv) Antimycin A (ANTI A) and Rotenone (ROT) 2 µM each were sequentially performed for 3 measurement cycles each.

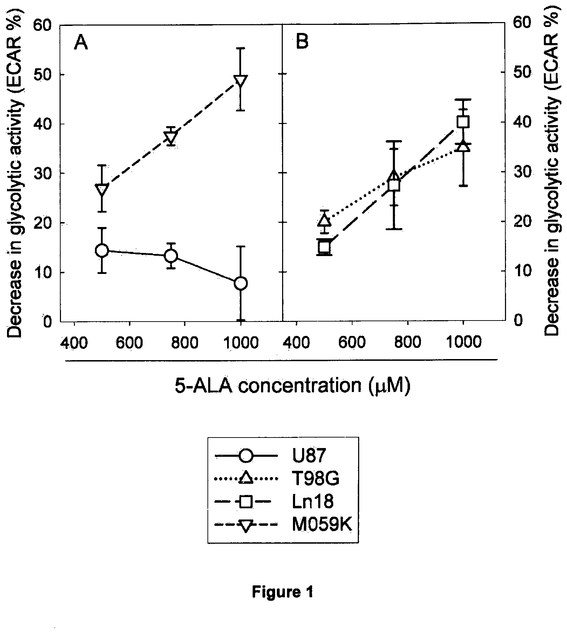

It was found that addition of 5-ALA conferred a drop in the glycolytic activity of those cell lines which was dose dependent, as shown in Figure 1 (Error bars represent one standard deviation (SD)).

### Example 2

The ATP contributions of mitochondrial respiration and cell glycolysis in U87, T98G, M059K and LN18 human GBM cells in the presence or absence of 5-ALA, were deconvoluted quantified by use of the Seahorse XF Real-Time ATP Rate Assay Kit. In brief the same protocol as in Example 1 was followed without the injection of FCCP and with only three basal measurement cycles, while the corresponding final concentrations of OLIGO and ROT/ANTI A were this time 1.5 and 0.5 µM. The ATP rates were determined with use of the Agilent Seahorse XF Real-Time ATP Rate Assay Report Generator.

The wells in the bottom row of the seahorse plates were in all cases left devoid of cells and were used as blank values. These were automatically subtracted by the Seahorse analyser software.

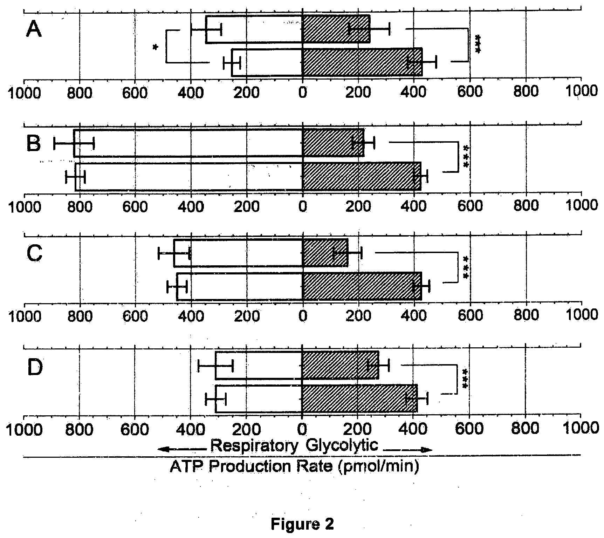

The changes in ATP production from either respiration or glycolysis were evaluated upon addition of 1000µM of 5-ALA. The results are presented in Figure 2 (Error bars represent one SD. * P < 0.05, **P < 0.01, ***P < 0.001). In Figure 2, A shows the results for U87, B for T98G, C for M059K and D for LN18 cell line. The higher bars are the results of 5-ALA and the lower bars are the results of the control.

For all the cell lines studied there was either no change or an increase in ATP production from respiration. In fact, the mitochondrial respiration-derived ATP showed a marginally significant increase in U87 cells upon introduction of 5-ALA (~100pmole/min)). In all the cases, however, the drop in glycolytic ATP production upon introduction of 5-ALA, was profound. The reductions registered upon introduction of 5-ALA to the cells were ∼ -200 pmole/min for U87 and T98G, ~ -250 pmole/min for M059K and ∼ -140 pmole/min for LN18.

### Example 3

This Example shows the results of a spectrophotometric assessment of LDH kinetics with or without 5-ALA, in purified enzyme assays or cell lysates.

The reaction buffer used was a TRIZMA buffer (50 mM) with a final pH of 6.5. The reaction buffer also contained 20 mM of sodium pyruvate (the natural substrate for LDH) and 0.4 mg/mL NADH.

A. enzyme solutions. 0.5 mL of an aqueous LDH solution was mixed with 0.5 mL reaction buffer to start the LDH reduction of pyruvate. The final LDH concentration was always 5 µM. The decay absorbance kinetics of NADH oxidation to NAD+ corresponding to pyruvate conversion to L-lactic acid by LDH were in each case monitored at 340 nm (εNADH 340nm = 0.00622 Lµmol-1 cm-1), using a Shimadzu UV-2550 UV-VIS spectrophotometer (Shimadzu Corp., Kyoto, Japan). Absorption measurements were recorded every 2 sec for 2 min. In experiments with inhibitor, 5-ALA was added to and mixed with the aqueous solution of LDH, just before the addition of the reaction buffer to initiate the enzymatic reaction. The final concentration of 5-ALA in the total volume of the reaction mix (1 mL) was 5 mM for 5-ALA. In the enzymatic assays LDH refers to the LDH-A isoform.

All experiments were repeated independently at least 3 times.

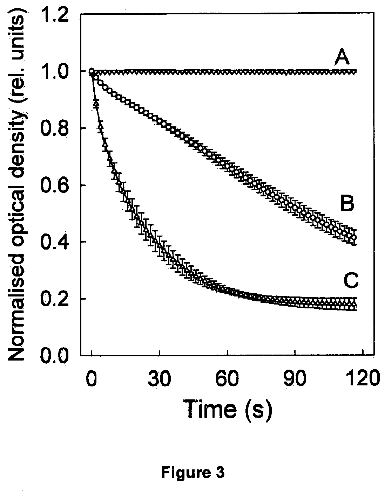

Figure 3 shows the results of LDH inhibition in enzyme solutions (Error bars represent one SD).

In order to ensure linearity, the initial slopes of the curves presented in Figure 3, for their first 5 data points, corresponding to 8 sec were determined. The slopes are shown in Table 1 and they correspond to the Vmax of the enzyme at the concentration studied. Upon addition of 5-ALA, the enzyme functioned at ~22% of its Vmax.

**Table 1**

| | Enzyme | Cell lysates | | | |
|---|---|---|---|---|---|
| | | U87 | T98G | Ln18 | M059K |
| No inhib. | -2.28 | -1.43 | -1.20 | -1.48 | -1.47 |
| +5-ALA | -0.51 | -0.32 | -0.57 | -0.47 | -0.53 |

In the second phase of the experiments, the enzymatic activity of LDH present in cell lysates was investigated.

Cell lysates were obtained from LN18, T98G, U87 and M059K GBM cells. In brief, cells from confluent T175 flasks were harvested with the use of TrypLE^{™} Express Enzyme (Gibco, REF# 12604021) and resuspended in ddH2O with Triton^{™} X-100 (0,25%). The cell lysate measurements were performed according to the the enzymatic solution measurents, however in this case, cell lysates equivalent to 106 cells were each time added to the H2O half of the reaction mix. The reaction buffer was then added to initiate the enzymatic reactions. 5-ALA was added to the final concentration of 0.75 mM, to the cell lysates prior to the addition of the reaction buffer.

All experiments were repeated independently at least 3 times.

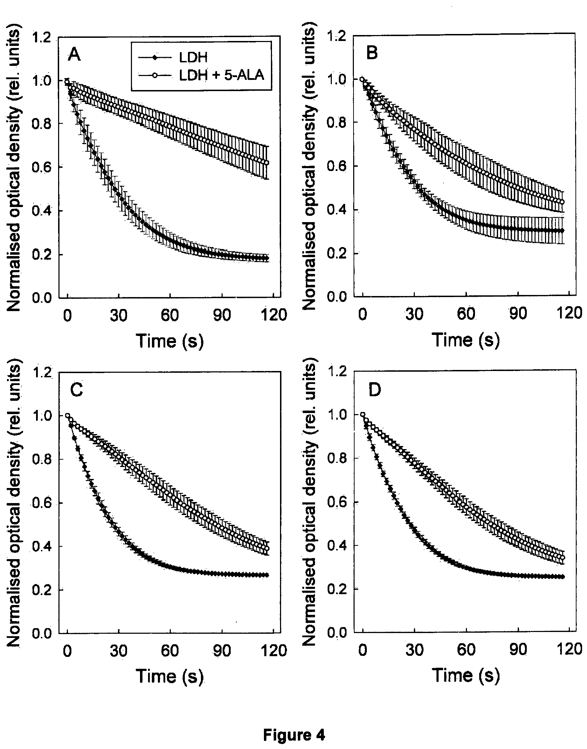

The results for all the cell lines studied are presented in Figure 4 (Error bars represent one SD). From these data it is evident that in the case of lysates, the tartronic acid-related curves (NADH absorbance) exhibited an initial rise, before starting to decay. Since this initial 'kink' was not evident in the enzymatic assays, it was attributed to spontaneous conversion of intracellular NAD+ to NADH, upon addition of the tartronic containing solution. The LDH Vmax values for the cell lysate volume studied, are also shown in Table 1 above. From these data it can be inferred that U87, LN18 and M059K contain similar amounts of LDH, ~ 19% more than T98G.

### Example 4

This example shows the effect of 5-ALA administration on the viability of three human GBM cell lines (LN18, U87 and T98G).

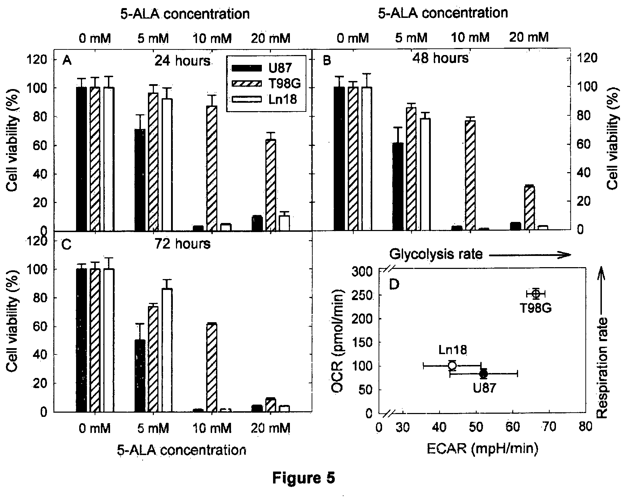

The cells were incubated with 5, 10 and 20 mM 5-ALA for three days and each day the effect of 5-ALA on their viability was assessed by standard MTT assays at 24, 48 and 72h following incubation. The results are shown in Figure 5 (Error bars represent one SD).

From the data in Figure 5 it can be seen that there is a strong reduction is cell viability at all measurement timepoints for LN18 and U87 cells. More specifically both U87 and LN18 cells show a marked reduction of about 90-98% in cell viability for all time points when incubated with 10mM 5-ALA, which is, in general, maintained also for incubation at the higher 5-ALA dose of 20 mM. T98G are not so sensitive to incubation with 10mM 5-ALA, reaching only LD40 after 72h incubation. Increase of the 5-ALA concentration to 20mM, however, caused a gradual drop in the viability of T98G to -30% at 48h and 5-10% at 72h. The corresponding metabolic profiles of the 3 cell lines investigated are presented in Figure 7D. Both U87 and LN18 seem to follow a Warburg metabolic phenotype, with reduced respiration rates, while T98G exhibit high metabolism with respect to both OCR and ECAR.

## Claims

1. A compound according to formula (I), or a pharmaceutically acceptable salt thereof 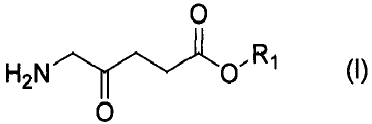 wherein R₁ is hydrogen, or a C₁-C₆ aklyl group,
for use in the treatment of cancer in a patient by inhibiting the activity of lactate dehydrogenase (LDH) in cancer cells.

2. The compound for use according to claim 1, wherein R₁ is hydrogen.

3. The compound for use according to claim 1 or 2, wherein the cancer is selected from brain cancer, sarcoma, including osteogenic and soft tissue sarcoma, breast cancer, lung cancer, cerebral cancer, bladder cancer, thyroid cancer, prostate cancer, colon cancer, rectum cancer, pancreatic cancer, stomach cancer, liver cancer, liver duct cancer, uterine cancer, hepatic cancer, renal cancer, prostate cancer, cervical cancer, ovarian cancer, lymphoma, including Hodgkin and non-Hodgkin lymphoma, neuroblastoma, melanoma, myeloma, Wilm's tumour, leukemia, including acute lymphoblastic leukaemia and acute myeloblastic leukaemia, astrocytoma, glioma, mixed glioma, retinoblastomas, or mesothelioma.

4. The compound for use according to any one of the preceding claims, wherein the cancer is selected from glioma, astrocytoma, glioblastoma multiforme, mixed glioma, pancreas cancer, liver cancer, liver duct cancer, cervical cancer, mesothelioma, colon cancer, or lung cancer.

5. The compound for use according to any one of the preceding claims, wherein the cancer is selected from glioma, astrocytoma, or glioblastoma multiforme.

6. The compound for use according to any one of the preceding claims, wherein the treatment comprises sustained delivery of the compound of formula (I) or a pharmaceutically acceptable salt thereof to the patient.

7. The compound for use according to claim 6, wherein the duration of the sustained delivery is from 12 hours to one month.

8. The compound for use according to claim 6 or 7, wherein the duration of the sustained delivery is from 1 day to 20 days.

9. The compound for use according to any one of claims 6 or 8, wherein the delivered amount is such that the concentration of 5-aminolevulinic acid in the patient's blood in the course of the treatment is from 0.05mM to 50mM.

10. The compound for use according to any one of claims 6 to 9, wherein the delivered amount is such that the concentration of 5-aminolevulinic acid in the patient's blood in the course of the treatment is from 0.1mM to 20mM.

11. A pharmaceutical composition comprising as active ingredient an LDH inhibitor for use in the treatment of cancer by inhibiting the activity of LDH in cancer cells, wherein the LDH inhibitor is a compound of formula (I) or a pharmaceutically acceptable salt thereof 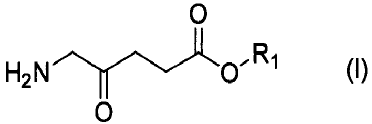 wherein R₁ is hydrogen, or a C₁-C₆ aklyl group.

12. The pharmaceutical composition for use according to claim 11, wherein R₁ is hydrogen.

13. The pharmaceutical composition for use according to claim 11 or 12, wherein the cancer is selected from brain cancer, sarcoma, including osteogenic and soft tissue sarcoma, breast cancer, lung cancer, cerebral cancer, bladder cancer, thyroid cancer, prostate cancer, colon cancer, rectum cancer, pancreatic cancer, stomach cancer, liver cancer, liver duct cancer, uterine cancer, hepatic cancer, renal cancer, prostate cancer, cervical cancer, ovarian cancer, lymphoma, including Hodgkin and non-Hodgkin lymphoma, neuroblastoma, melanoma, myeloma, Wilm's tumour, leukemia, including acute lymphoblastic leukaemia and acute myeloblastic leukaemia, astrocytoma, glioma, mixed glioma, retinoblastomas, or mesothelioma.

14. The pharmaceutical composition for use according to any one of claims 11 to 13, wherein the cancer is selected from glioma, astrocytoma, or glioblastoma multiforme.

15. The pharmaceutical composition for use according to any one of claims 11 to 14, wherein the composition is a sustained release composition.
